# EUROPEAN PATENT SPECIFICATION

(11) **EP 1 328 528 B1**
(45) Date of publication and mention of the grant of the patent: **29.12.2004**
(21) Application number: 01987751.3
(22) Date of filing: 18.10.2001
(51) Int. Cl.: C07D 503/02, C07D 503/08, C12P 17/18, A61K 31/43

(54) **Pharmaceutical compositions comprising clavulanic acid**
Pharmazeutische Zubereitungen enthaltend Clavulansäure
Compositions pharmaceutiques contenant d'acide clavulanique

(30) Priority: 20.10.2000 AT 18062000
(43) Date of publication of application: 23.07.2003
(73) Proprietor: SANDOZ AG, 4002 Basel (CH)
(72) Inventor: HORKOVICS-KOVATS, Stefan, A-6300 Angath (AT); RANEBURGER, Johannes, A-6300 Wörgl (AT); SCHWARZ, Franz, Xaver, A-6300 Wörgl (AT)
(74) Representative: Gros, Florent
(86) International application number: PCT/EP2001/012076
(87) International publication number: WO 2002/032906

(56) References cited:
- WO-A-00/41478
- WO-A-97/18216
- WO-A-98/23622
- US-A- 4 223 006

## Description

The present invention relates to pharmaceutical composition, such as pharmaceutical compositions of clavulanic acid in combination with amoxicillin.

AUGMENTIN®, see e.g. Merck Index 12th Edition, Item 2402, is a commercial form of pharmaceutical compositions of the β-lactamase inhibitor clavulanic acid (in the form of the potassium salt) in combination with the broad-spectrum antibiotic amoxicillin (in the form of the trihydrate). When orally administered, the active ingredient amoxicillin and the β-lactamase inhibitor of such compositions should be available in dissolved form at the resorption site in reasonable amounts and concentrations.

According to methods as conventional in the preparation of clavulanic acid, clavulanic acid and its salts may be obtained, e.g. in the form of needles, rod-shaped crystals or rosettes; in a grain size and grain size distribution such, that 80% of the particles, e.g. crystalline particles, have a grain size of 40 µm and more, e.g. up to 250 µm and more, and the median of the particles is of 70 µm and more, e.g. up to 110 µm and more. The grain size and distribution of grain size may be determined by known methods, e.g. by pattern analysis or laser diffraction processes. The median is a central value that can be used instead of an average value. In the case of grain size, the median means that the actual grain size of 50% of the particles is larger and of 50% of the particles is smaller than the median. The median may be determined by a method as conventional, e.g. by numeric calculation based on the actual grain size distribution.

We have now surprisingly found that clavulanic acid may be resorbed particularly well from the gastrointestinal tract following oral administration if the median, and/or the grain size, of the clavulanic acid particles, are of a certain size.

In one aspect, the present invention provides clavulanic acid, e.g. in the form of a potassium salt, in which
- the median of the particles is between 8 µm, preferably 10 µm, and 35 µm, preferably 30 µm, and/or in which
- 80% of the particles of the clavulanic acid have a grain size of 1 µm to 70 µm, such as 2 µm to 70 µm.

The median is preferably from 10 µm to 30 µm, more preferably from 12 µm to 30 µm. Preferably 80% of the particles of the clavulanic acid have a grain size of 2 µm to 70 µm. Clavulanic acid includes clavulanic acid in free form and clavulanic acid in the form of a salt, e.g. a pharmaceutically acceptable salt, such as an alkali or alkaline earth salt, preferably a potassium salt. Clavulanic acid according to the present invention is in the form of solid particles, e.g. in powder or crystalline form.
It has surprisingly also been found that clavulanic acid particles according to the present invention keeps its favourable resorption characteristics, if administered in the form of pharmaceutical compositions, e.g. AUGMENTIN® compositions.

In another aspect, the present invention provides a pharmaceutical composition containing clavulanic acid, in which
- the median of the clavulanic acid particles is from 8 µm, preferably from 10 µm, to 35 µm, preferably to 30 µm; and/or in which
- 80% of the clavulanic acid particles, e.g. crystalline particles or powder particles, of the clavulanic acid, have a grain size of 1 µm to 70 µm, such as 2 µm to 70 µm.

A pharmaceutical composition includes oral pharmaceutical compositions, such as tablets, film-coated tablets, chewing tablets, powders for oral suspensions and dispersible tablets.

Clavulanic acid according to the present invention may be obtained e.g. as follows:
Clavulanic acid, preferably in the form of a potassium salt, may be isolated, e.g. crystallised, from a solvent, preferably n-butanol and/or isobutanol, and the crystals obtained, e.g. still moist with solvent, are treated in a combined drier/mixer until the median of the particles is from 8 µm to 35 µm, and/or 80% of the clavulanic acid particles have a grain size of 1 µm to 70 µm.
Surprisingly we have found that, if n-butanol and/or isobutanol is used as a solvent in clavulanic acid (potassium salt) preparation, the clavulanic acid particles may be produced in a size which is particularly useful for obtaining clavulanic acid according to the present invention by following treatment in a combined drier and mixer compared with solvents other than n-butanol and/or isobutanol.

In another aspect, the present invention provides a process for the production of clavulanic acid in which the median of particles is between 8 µm and 35 µm, and/or in which 80% of the clavulanic acid particles have a grain size of 1 µm to 70 µm, comprising the steps
a) isolating clavulanic acid, e.g. in the form of a potassium salt, from organic solvent, e.g. n-butanol and/or isobutanol, and
b) treating the particles obtained in step (a) in a combined drier/mixer until the median of the particles is between 8 µm and 35 µm, and/or until 80% of the clavulanic acid particles have a grain size of 1 µm to 70 µm.

Isolation of clavulanic acid in the form of a pharmaceutically acceptable salt from n-butanol and/or isobutanol is known and is described e.g. in WO 97/18216. The content of WO 97/18216 and the content of the literature cited therein are incorporated in the present application by reference as a part of the present application.
In WO 97/18216, a process is described, according to which clavulanic acid in n-butanol and/or isobutanol as the solvent is converted into a pharmaceutically acceptable salt of clavulanic acid, e.g. an alkali or alkaline earth salt, preferably a potassium salt. As a starting material, clavulanic acid may be used as such or in the form of a salt, e.g. a lithium salt or an amine salt, preferably an amine salt. Amine salts include salts of clavulanic acid with an amine as disclosed in WO 97/18216, e.g. tert.-butylamine, tert.-octylamine (2-amino-2,4,4-trimethylpentane), N,N'-diisopropylethylenediamine, N,N,N',N'-tetramethyl-diaminoethane and 1,3-bis(dimethylamino)-2-propanol, preferably tert.-octylamine or tert.butylamine. Clavulanic acid in the form of a salt with an amine may be produced e.g. according to one of the methods disclosed in WO 97/18216 including the literature cited therein, preferably as follows:
a) Fermentating an appropriate micro-organism, e.g. a micro-organism which is capable of producing clavulanic acid during fermentation, to obtain an impure aqueous fermentation broth containing clavulanic acid;
b) optionally harvesting a part of the fermentation broth obtained in step a),
c) optionally pre-purifying a fermentation broth of step a) or b), e.g. by
   - removing at least part of the solids from the fermentation broth, and/or
   - extracting an impure or pre-purified aqueous fermentation broth containing clavulanic acid, with an organic solvent which is able to form two phases when in contact with water;
   to obtain a pre-purified aqueous fermentation broth or aqueous solution containing clavulanic acid;
d) optionally concentrating an impure or pre-purified fermentation broth or aqueous solution of step a), b) or c);
e) acidifying a fermentation broth of step a), b), c) or d) to obtain an acidified impure or pre-purified, optionally concentrated, aqueous fermentation broth or solution containing clavulanic acid;
f) extracting an acidified fermentation broth or solution of step e) with an organic solvent in which clavulanic acid is soluble under the conditions of extraction, and which is able to form two phases when in contact with water, to obtain a solution of clavulanic acid in an organic solvent;
g) treating a solution obtained in step f) with an amine, preferably tert.-butylamine, tert.-octylamine, N,N'-diisopropytethylenediamine, N,N,N',N'-tetramethyl-diaminoethane or 1,3-bis(dimethylamino)-2-propanol, more preferably tert.-octylamine or tert.-butylamine, to obtain clavulanic acid in the form of a salt with an amine, and/or in the form of a solvate, such as an acetone solvent; and optionally isolating the salt obtained; and
h) converting a salt of step g) into a pharmaceutically acceptable salt of clavulanic acid, e.g. a potassium salt.

Conversion according to step h) may be carried out as appropriate, e.g. as follows:
Clavulanic acid in the form of a salt with an amine is dissolved in n-butanol and/or isobutanol. It is preferable to use either n-butanol or isobutanol, e.g. in an amount that is sufficient to dissolve the clavulanic acid. Water, e.g. 0.5 to 10%, e.g. 1.0 to 5%, such as 1.0 to 4%, e.g. 1.5 to 3.0%, may be present in the solution. The solution obtained is optionally treated with activated carbon, and is brought into contact with a source of cation which is capable of forming a pharmaceutically acceptable salt with clavulanic acid. Cation sources of this kind are described in WO 97/18216, for example in literature cited therein, and include e.g. alkaline earth or alkali salts of a (C₂₋₈)-carboxylic acid, e.g. 2-ethylhexanoic acid, for example the potassium salt thereof, as well as acetates, e.g. potassium acetate. If an acetate is used as a source of cation, acetic acid may be additionally added to the reaction mixture. Contact of the cation source with the solution of an amine salt of clavulanic acid may take place according to one of the methods disclosed in WO 97/18216, for example in literature cited therein, and is preferably effected as follows:
A solution of the cation source in a solvent, preferably in n-butanol and/or isobutanol, is added, e.g. in portions, to a solution of an amine salt of clavulanic acid. At least one equivalent of the cation source, preferably 1.0, such as about 1.1, to preferably 3.0, such as about 2.0 equivalents, are used per mol of clavulanic acid (salt). Clavulanic acid in the form of a pharmaceutically acceptable salt may precipitate from the reaction mixture. For example, in order to complete precipitation, a further solvent in which the pharmaceutically acceptable salt is poorly soluble may be added to the mixture, and/or the mixture obtained may be cooled e.g. to temperatures of below 0°C to about 10°C, such as about 0°C to about 5°C. The pharmaceutically acceptable salt of clavulanic acid is isolated, e.g. by filtration, centrifugation, and is obtained in solid form, e.g. in crystalline form, either rosette-free or in the form of rosettes.
The pharmaceutically acceptable salt of clavulanic acid, e.g. whilst moist with solvent, is dried in a drier which is combined with a mixer, whereby the shearing force of the mixer is set such that clavulanic acid is obtained, in which the median of the particles is from 8 µm to 35 µm and/or 80% of the particles, e.g. crystal or powder particles, of the clavulanic acid have a grain size of 1 µm to 70 µm. Driers combined with a mixer are commercially available. The shearing force of a mixer and thus the particle size of the clavulanic acid may be regulated by switching on the mixer for a shorter or longer period during drying. The most suitable lengths of time that the mixer should be switched on and off may be determined by preliminary tests.
Clavulanic acid according to the present invention, in the form of a pharmaceutically acceptable salt in which the median of the particles is from 8 µm to 35 µm, and/or in which 80% of the particles, e.g. crystal or powder particles, of the clavulanic acid have a grain size of 1 µm to 70 µm, may be obtained.

In another aspect, the present invention provides a process for the preparation of clavulanic acid in the form of a pharmaceutically acceptable salt, such as a potassium salt, in which the median of the particles is from 8 µm to 35 µm and/or in which 80% of the clavulanic acid particles have a grain size of 1 µm to 70 µm, said process comprising the steps
a) converting an amine salt of clavulanic acid into a pharmaceutically acceptable salt of clavulanic acid, e.g. wherein
   - said amine salt is obtained by treating a solution of clavulanic acid in an organic solvent with an amine, e.g. wherein
   - said solution of clavulanic acid in an organic solvent is obtained by extracting an acidified aqueous, impure or pre-purified fermentation broth or solution containing clavulanic acid with an organic solvent in which clavulanic acid is soluble under the conditions of extraction, and which is able to form two phases when in contact with water, e.g. wherein
   - said acidified impure or pre-purified fermentation broth or solution containing clavulanic acid is obtained by acidifying an optionally pre-concentrated aqueous, impure or pre-purified fermentation broth or solution containing clavulanic acid, e.g. wherein
   - said optionally pre-concentrated aqueous, impure or pre-purified fermentation broth or solution containing clavulanic acid is obtained by optionally concentrating an impure or pre-purified fermentation broth or aqueous solution containing clavulanic acid, e.g. wherein
   - said pre-purified aqueous fermentation broth or aqueous solution containing clavulanic acid is obtained by removing at least part of the solids from a fermentation broth containing caivulanic acid, and/or by extracting an impure or pre-purified aqueous fermentation broth containing clavulanic acid, with an organic solvent which is able to form two phases when in contact with water; e.g. wherein
   - said impure aqueous fermentation broth containing clavulanic acid is obtained by fermentating an appropriate micro-organism, and
b) treating the pharmaceutically acceptable salt of clavulanic acid obtained in a), e.g. in a form which is moist with solvent, in a combined drier and mixer until the median of the particles is from 8 µm to 35 µm and/or until 80% of the particles have a grain size of 1 µm to 70 µm, e.g. the shearing force of the mixer is used to adjust the appropriate median and/or grain size, e.g. by appropriate switching the mixer on and off during drying.

An aqueous solution of clavulanic acid may also be produced, e.g. by dissolving clavulanic acid in an aqueous solvent system.

In the following examples, all temperatures are in degree Centigrade and are uncorrected.

The following abbreviations are used:
CS-K: Clavulanic acid in the form of a potassium salt

### Example A

CS-K is produced according to a method of examples 1 to 9 of WO 97/18216 and is isolated from the reaction mixture. Crystalline particles having a median greater than 35 µm and wherein less than 80% of the particles have a grain size between 1 µm and 70 µm are obtained. CS-K thus obtained is treated whilst moist with solvent, according to any of the following examples:

### Example 1

CS-K obtained according to example A, in which the median of the particles is 103 µm and in which 80% of the particles have a grain size of 40 µm to 250 µm (hereinafter referred to as "CS-B"), in a form which is moist with solvent, is treated in a drier in which the particles can be simultaneously mixed and dried. The mixer is switched on and off during drying, so that CS-K particles are obtained in which the median of the particles is 25 µm and in which 80% of the particles have a grain size of 5 µm to 60 µm (hereinafter referred to as "CS-A"). The number of times to switch the mixer on and off and the mixing time to obtain CS-A is determined in a preliminary test.

### Example 2

CS-K obtained according to example A, in which the median of the particles is 87 µm and in which 80% of the particles have a grain size of 50 µm to 200 µm (hereinafter referred to as "CS-D") is treated as described in example 1 but using different mixing times and mixing switches. CS-K particles are obtained in which the median of the particles is 18 µm and in which 80% of the particles have a grain size of 2 µm to 50 µm (hereinafter referred to as "CS-C").

### Example 3

### Film-coated tablets

Amoxicillin in the form of a trihydrate and cross-linked starch are mixed, granulated with water, dried and equalised. The granulate obtained is mixed with CS-A or CS-B, cellulose, talcum and magnesium stearate, and the mixture obtained is pressed into tablets.
Tablets "A" or "B" are obtained, which contain per tablet 1.0 g of amoxicillin in the form of a trihydrate (corresponds to 0.875 g of amoxicillin), cross-linked starch, cellulose, talcum and magnesium stearate and
- in tablet "A" 0.149 g of "CS-A" (corresponds to 0.125 g of clavulanic acid), and
- in tablet "B" 0.149 g of "CS-B" (corresponds to 0.125 g of clavulanic acid).
Tablets "A" and "B" are coated with a film composition. Film-coated tablets "A" or film-coated tablets "B" are obtained, which comprise of tablets "A" or "B", and which are coated per tablet with a film coating comprising of film-forming components, pigments, plasticisers.

### Example 4

### Chewing tablets

Amoxicillin in the form of a trihydrate, sugar alcohols and colourant are mixed, the mixture obtained is granulated with water, dried and equalised. The granulate obtained is mixed with CS-C or CS-D, sweeteners, aromatizers, cross-linked starch, talcum and magnesium stearate, and the mixture obtained is pressed into tablets. Chewing tablets "C" or "D" are obtained, which contain per tablet 0.466 g of amoxicillin in the form of a trihydrate (corresponds to 0.4 g of amoxicillin), sugar alcohols, sweeteners, aromatic substances, cross-linked starch, talcum and magnesium stearate and
- in Chewing tablet "C" 0.068 g of CS-C (corresponds to 0.057 g of clavulanic acid); and
- in Chewing tablet "D" 0.068 g of CS-D (corresponds to 0.057 g of clavulanic acid).

### Example 5

### Powders for oral suspensions

Amoxicillin in the form of a trihydrate, CS-C or CS-D, carboxylic acids, sweeteners, aromatizers, thickeners, sugar alcohols and silicon dioxide are admixed. Powders for oral suspensions "C" or "D" are thereby obtained. 23 g portions of the obtained powders for oral suspensions "C" or "D" are filled into glass bottles (multi-dose containers). Prior to administration, 84 ml of water is added to this bottle. 100 ml of a ready-to-use, oral suspension "C" or "D" is obtained, representing 20 doses each of 5 ml. Each dose contains 0.466 g of amoxicillin in the form of a trihydrate (corresponding to 0.4 g of amoxicillin), carboxylic acids, sweeteners, aromatizers, thickeners, sugar alcohols, silicon dioxide and
- in oral suspension "C" 0.068 g of CS-C (corresponds to 0.057 g of clavulanic acid);
- in oral suspension "D" 0.068 g of CS-D (corresponds to 0.057 g of clavulanic acid).

### Testing for bioavailability

To 24 healthy probands (test persons) are administered
- a film-coated tablet "A" or a film-coated tablet "B"
- a chewing tablet "C" or a chewing tablet "D"
- an oral suspension "C" or an oral suspension "D".
The bioavailability of clavulanic acid in the administered medicinal forms is determined in accordance with the guidelines of the European Agency for the Evaluation of Medicinal Products (EMEA), Human Medicines Evaluation Unit, Draft of the Committee for proprietary Medicinal Products (CPMP) under the title "Note for guidance on the investigation of bioavailability and bioequivalence" of 17-Dec-1998 (CPMP/EWP/QWP/1401/98), in keeping with the relevant EU and ICH Guidelines and Regulations.

Cₘₐₓ is the maximum plasma concentration.
AUC is the area under the plasma concentration curve, extrapolated to t = infinite.

The AUC (µg x h/ml) and Cₘₐₓ (µg/ml) values indicated in TABLE 1 below are obtained for each respective pharmaceutical composition used:

**TABLE 1**

| **Medicinal form** | **AUC (µg x h/ml)** | **C**_{**max**} **(µg/ml)** |
|---|---|---|
| film-coated tablets "A" | 6.6+/-2.3 | 3.1+/-1.1 |
| film-coated tablets "B" | 6.0 +/- 2.3 | 2.7+/-1.1 |
| chewing tablets "C" | 3.0+/-0.85 | 1.45+/-0.4 |
| chewing tablets "D" | 2.6 +/- 0.83 | 1.3+/-0.4 |
| oral suspension "C" | 3.2+/-1.0 | 1.6+/-0.4 |
| oral suspension "D" | 2.8 +/- 1.1 | 1.4+/-0.5 |

From TABLE 1, it is immediately evident that the bioavailability of clavulanic acid in film-coated tablets "A", chewing tablets "C" and oral suspensions "C", which contain CS-K particles having a median = 25 µm, 80% grain size of 5 µm to 60 µm, or a median = 18 µm, 80% grain size of 2 µm to 50 µm, respectively, is improved, for at least 10%, compared with film-coated tablets "B", chewing tablets "D" and oral suspensions "D", which contain CS-K particles having a median = 103 µm and 80% grain size of 40 µm to 250 µm, or a median = 87 µm and 80% grain size of 50 µm to 200 µm, respectively.

## Claims

1. Clavulanic acid or a salt thereof in the form of particles of which the median is from 8 µm to 35 µm.

2. Clavulanic acid or a salt thereof in the form of particles according to claim 1, wherein 80% of the particles have a grain size of 1 µm to 70 µm.

3. Clavulanic acid or a salt thereof in the form of particles, whereof
- 80% have a grain size in the range of 2 µm to 70 µm and
- the median is between 10 µm and 30 µm.

4. Clavulanic acid in the form of particles according to any one of claims 1 to 3, wherein the clavulanic acid is in the form of a potassium salt.

5. A pharmaceutical composition comprising clavulanic acid or a salt thereof in the form of particles as defined in any one of claims 1 to 4.

6. A pharmaceutical composition according to claim 5, which is a tablet, film-coated tablet, chewing tablet, powder for oral suspensions, or dispersible tablet.

7. A process for the production of clavulanic acid in the form of particles as defined in any one of claims 1 to 4, comprising the steps of
a) converting clavulanic acid into a pharmaceutically acceptable salt of clavulanic acid in n-butanol and/or isobutanol as a solvent,
b) isolating the salt obtained in step a) from the solvent, and
c) treating the particles obtained in step b) in a combined drier/mixer until the median of the particles is between 8 µm and 35 µm.

8. A process according to claim 7, wherein the pharmaceutically acceptable salt is a potassium salt.

9. A process for the production of clavulanic acid or a salt thereof in the form of particles as defined in any one of claims 1 to 4, comprising
a) fermentating an appropriate micro-organism to obtain an impure aqueous fermentation broth containing clavulanic acid;
b) optionally harvesting a part of the fermentation broth obtained in step a),
c) optionally pre-purifying a fermentation broth of step a) or b), to obtain a pre-purified aqueous fermentation broth or aqueous solution containing clavulanic acid;
d) optionally concentrating an impure or pre-purified fermentation broth or aqueous solution of step a), b) or c);
e) acidifying a fermentation broth of step a), b), c) or d) to obtain an acidified impure or pre-purified, optionally concentrated, aqueous fermentation broth or solution containing clavulanic acid;
f) extracting an acidified fermentation broth or solution of step e) with an organic solvent in which clavulanic acid is soluble under the conditions of extraction, and which is able to form two phases when in contact with water, to obtain a solution of clavulanic acid in an organic solvent;
g) treating the solution obtained in step f) with an amine, to obtain clavulanic acid in the form of a salt with an amine,
h) converting a salt of step g) into a pharmaceutically acceptable salt of clavulanic acid in n-butanol and/or isobutanol as a solvent,
i) isolating the salt obtained in step h) from the solvent, and
j) treating the particles obtained in step i) in a combined drier/mixer until the median of the particles is between 8 µm and 35 µm.

## Patentansprüche

1. Clavulansäure oder ein Salz hiervon in Form von Teilchen, von denen der Mittelwert (Mediane) von 8 µm bis 35 µm reicht.

2. Clavulansäure oder ein Salz hiervon in Form von Teilchen nach Anspruch 1, worin 80 % der Teilchen eine Korngröße von 1 µm bis 70 µm haben.

3. Clavulansäure oder ein Salz hiervon in Form von Teilchen, wovon
- 80 % eine Korngröße im Bereich von 2 µm bis 70 µm haben und
- der Mittelwert (Mediane) zwischen 10 µm und 30 µm liegt.

4. Clavulansäure in Form von Teilchen nach einem der Ansprüche 1 bis 3, worin die Clavulansäure in Form eines Kaliumsalzes vorliegt.

5. Pharmazeutische Zusammensetzung, enthaltend Clavulansäure oder ein Salz hiervon in Form von Teilchen gemäß Definition nach einem der Ansprüche 1 bis 4.

6. Pharmazeutische Zusammensetzung nach Anspruch 5, welche eine Tablette, eine filmbeschichtete Tablette, eine Kautablette, ein Pulver für orale Suspensionen oder eine dispersible Tablette ist.

7. Verfahren zur Herstellung von Clavulansäure in Form von Teilchen gemäß Definition nach einem der Ansprüche 1 bis 4, umfassend die Schritte einer
a) Umwandlung von Clavulansäure in ein pharmazeutisch annehmbares Satz von Clavulansäure in n-Butanol und/oder Isobutanol als Lösemittel,
b) Isolierung des im Schritt a) erhaltenen Salzes aus dem Lösemittel und
c) Behandlung der im Schritt b) erhaltenen Teilchen in einem kombinierten Trockner/Mischer bis der Mittelwert (Mediane) der Teilchen zwischen 8 µm und 35 µm liegt.

8. Verfahren nach Anspruch 7, worin das pharmazeutisch annehmbare Salz ein Kaliumsalz ist.

9. Verfahren zur Herstellung von Clavulansäure oder eines Salzes hiervon in Form von Teilchen gemäß Definition nach einem der Ansprüche 1 bis 4, umfassend eine
a) Fermentation eines geeigneten Mikroorganismus unter Erhalt einer unreinen wässrigen Fermentationsbrühe, die Clavulansäure enthält,
b) optionale Erntung eines Teils der im Schritt a) enthaltenen Fermentationsbrühe,
c) optionale Vorreinigung einer Fermentationsbrühe von Schritt a) oder Schritt b) unter Erhalt einer vorgereinigten wässrigen Fermentationsbrühe oder wässrigen Lösung, die Clavulansäure enthält,
d) optionale Konzentration einer unreinen oder vorgereinigten Fermentationsbrühe oder wässrigen Lösung von Schritt a), b) oder c),
e) Ansäuerung einer Fermentationsbrühe von Schritt a), b), c) oder d) unter Erhalt einer angesäuerten unreinen oder vorgereinigten optional konzentrierten wässrigen Fermentationsbrühe oder Lösung, die Clavulansäure enthält,
f) Extraktion einer angesäuerten Fermentationsbrühe oder Lösung von Schritt e) mit einem organischen Lösemittel, worin die Clavulansäure unter den Bedingungen einer Extraktion löslich ist und das zur Bildung von zwei Phasen im Kontakt mit Wasser befähigt ist, unter Bildung einer Lösung von Clavulansäure in einem organischen Lösemittel,
g) Behandlung der gemäß Schritt f) erhaltenen Lösung mit einem Amin unter Erhalt von Clavulansäure in Form eines Salzes mit einem Amin,
h) Umwandlung eines Salzes von Schritt g) in ein pharmazeutisch annehmbares Salz von Clavulansäure in n-Butanol und/oder Isobutanol als Lösemittel,
i) Isolierung des im Schritt h) erhaltenen Salzes aus dem Lösemittel und
j) Behandlung der im Schritt i) erhaltenen Teilchen in einem kombinierten Trockner/Mischer bis der Mittelwert (Mediane) der Teilchen zwischen 8 µm und 35 µm liegt.

## Revendications

1. Acide clavulanique ou un de ses sels, sous forme de particules dont la médiane est de 8 µm à 35 µm.

2. Acide clavulanique ou un de ses sels, sous forme de particules selon la revendication 1, dans lequel 80 % des particules ont une taille de grain de 1 µm à 70 µm.

3. Acide clavulanique ou un de ses sels, sous forme de particules, dont
- 80 % ont une taille de grain située dans l'intervalle de 2 µm à 70 µm, et
- la médiane est comprise entre 10 µm et 30 µm.

4. Acide clavulanique sous forme de particules selon l'une quelconque des revendications 1 à 3, dans lequel l'acide clavulanique est sous forme d'un sel de potassium.

5. Composition pharmaceutique comprenant de l'acide clavulanique ou un de ses sels, sous forme de particules telles que définies dans l'une quelconque des revendications 1 à 4.

6. Composition pharmaceutique selon la revendication 5, qui est un comprimé, un comprimé enrobé d'un film, un comprimé à croquer, une poudre pour suspensions orales, ou un comprimé dispersable.

7. Procédé de production d'acide clavulanique sous forme de particules telles que définies dans l'une quelconque des revendications 1 à 4, comprenant les étapes consistant à :
a) transformer un acide clavulanique en un sel pharmaceutiquement acceptable de l'acide clavulanique dans du n-butanol et/ou de l'isobutanol comme solvant,
b) isoler le sel obtenu dans l'étape a) du solvant, et
c) traiter les particules obtenues dans l'étape b) dans un séchoir et un mélangeur combinés jusqu'à ce que la médiane des particules soit comprise entre 8 µm et 35 µm.

8. Procédé selon la revendication 7, dans lequel le sel pharmaceutiquement acceptable est un sel de potassium.

9. Procédé de production d'acide clavulanique ou d'un de ses sels, sous forme de particules telles que définies dans l'une quelconque des revendications 1 à 4, comprenant les étapes consistant à :
a) faire fermenter un micro-organisme approprié pour obtenir un bouillon de fermentation aqueux impur contenant de l'acide clavulanique ;
b) recueillir facultativement une partie du bouillon de fermentation obtenu dans l'étape a),
c) pré-purifier facultativement un bouillon de fermentation de l'étape a) ou b), pour obtenir un bouillon de fermentation aqueux pré-purifié ou une solution aqueuse contenant de l'acide clavulanique ;
d) concentrer facultativement un bouillon de fermentation impur ou pré-purifié ou une solution aqueuse de l'étape a), b) ou c) ;
e) acidifier un bouillon de fermentation de l'étape a), b), c) ou d) pour obtenir un bouillon de fermentation aqueux acidifié, impur ou pré-purifié, facultativement concentré, ou une solution contenant de l'acide clavulanique ;
f) extraire un bouillon de fermentation acidifié ou une solution de l'étape e) avec un solvant organique dans lequel l'acide clavulanique est soluble dans les conditions d'extraction, et qui est capable de former deux phases lorsqu'il entre en contact avec l'eau, pour obtenir une solution d'acide clavulanique dans un solvant organique ;
g) traiter la solution obtenue dans l'étape f) avec une amine, pour obtenir de l'acide clavulanique sous forme d'un sel avec une amine ;
h) transformer un sel de l'étape g) en un sel pharmaceutiquement acceptable de l'acide clavulanique dans du n-butanol et/ou de l'isobutanol comme solvant ;
i) isoler le sel obtenu dans l'étape h) du solvant, et
j) traiter les particules obtenues dans l'étape i) dans un séchoir et un mélangeur combinés jusqu'à ce que la médiane des particules soit située entre 8 µm et 35 µm.
